# EUROPÄISCHE PATENTSCHRIFT

(11) **EP 2 267 104 B1**
(45) Veröffentlichungstag und Bekanntmachung des Hinweises auf die Patenterteilung: **04.04.2018**
(21) Anmeldenummer: 10180585.1
(22) Anmeldetag: 21.02.2007
(51) Int. Cl.: C10L 1/2383, C10L 1/233, C10L 1/238, C10L 1/222, C10L 10/18, C10L 1/24, C10M 133/48, C08G 61/12, C10M 149/12, C10M 159/12, C07C 217/58, C08K 5/3437

(54) **Verwendung von mehrkernigen phenolischen Verbindungen als Dispergatoren**
Use of polynuclear phenolic compounds as dispersants
Utilisation de composés phénoliques polynucléaires comme dispersants

(30) Priorität: 27.02.2006 EP 06003970
(43) Veröffentlichungstag der Anmeldung: 29.12.2010
(62) Teilanmeldung aus: 07726453.9
(73) Patentinhaber: BASF SE, 67056 Ludwigshafen am Rhein (DE)
(72) Erfinder: Lange, Arno, 67098, Bad Dürkheim (DE); Mach, Helmut, 69115, Heidelberg (DE); Rath, Hans Peter, 67269, Grünstadt (DE); Posselt, Dietmar, 69120, Heidelberg (DE)
(74) Vertreter: BASF IP Association

(56) Entgegenhaltungen:
- WO-A-01/25293
- WO-A1-92/20762
- US-A- 2 459 112
- US-A- 3 413 347
- US-A- 4 038 043
- GILLIAN R. GOWARD ET AL: "Benzoxazine Oligomers: Evidence for a Helical Structure from Solid-State NMR Spectroscopy and DFT-Based Dynamics and Chemical Shift Calculations", JOURNAL OF THE AMERICAN CHEMICAL SOCIETY, Bd. 125, Nr. 19, 22. April 2003 (2003-04-22), Seiten 5792-5800, XP055128804, ISSN: 0002-7863, DOI: 10.1021/ja029059r

## Beschreibung

Die vorliegende Erfindung betrifft die Verwendung von speziellen mehrkernigen phenolischen Verbindungen als Dispergatoren zur Verhinderung von Ablagerungen im Kraftstoffsystem und/oder Verbrennungssystem von Turbinen. Weiterhin betrifft die vorliegende Erfindung eine Otto- und eine Dieselkraftstoffzusammensetzung, welche diese mehrkernigen phenolischen Verbindungen enthalten.

Die mechanischen, chemischen und/oder ästhetischen Eigenschaften von unbelebtem organischen Material, z. B. von Kunststoffen und Lacken, aber auch von Mineralölprodukten und Kraftstoffen, werden bekanntermaßen durch die Einwirkung von Licht, Sauerstoff und Wärme verschlechtert. Diese Verschlechterung zeigt sich üblicherweise als Vergilbung, Verfärbung, Rissbildung oder Versprödung des Materials. Es sind schon Stabilisatoren oder Stabilisatorzusammensetzungen bekannt, mit denen ein verbesserter Schutz gegen eine solche Beeinträchtigung von organischem Material durch Licht, Sauerstoff und Wärme erzielt werden kann.

So werden in der WO 05/073152 (1) 2-Alkyl-polyisobutenylphenole und deren Mannich-Addukte als Antioxidantien zur Stabilisierung von unbelebtem organischen Material gegen die Einwirkung von Licht, Sauerstoff und Wärme beschrieben. Als zu stabilisierende Materialien werden auch Kraftstoffe wie Ottokraftstoffe, Dieselkraftstoffe und Turbinenkraftstoffe sowie Schmierstoffzusammensetzungen genannt. In Turbinenkraftstoffen bewirken diese 2-Alkyl-polyisobutenyl-phenole und deren Mannich-Addukte eine Verbesserung der Thermostabilität sowie eine Verringerung der Ablagerungen im Kraftstoffkreislauf und Verbrennungssystem der Turbinen.

Auch die WO 03/106595 (2) offenbart neben hydrocarbylsubstituierten Bernsteinsäurederivaten und Polyalkenylthiophosphonatestern Mannich-Addukte aus hydrocarbylsubstituierten Phenolen, einem Aldehyd und einem Amin als Additive für Turbinenkraftstoffe (jet fuels) zur Verbesserung der Thermostabilität sowie zur Verringerung von Ablagerungen.

Tetrahydrobenzoxazine mit einem Benzolkern sind als Zusatzstoffe für Kraftstoff- und Schmierstoffzusammensetzungen bekannt. So offenbaren die WO 01/25293 (3) und die WO 01/25294 (4) derartige Tetrahydrobenzoxazine mit längerkettigen Resten wie Polyisobutenylresten, welche als Substituenten am Benzolkern sitzen, als ventilreinigende und ventilreinhaltende Ottokraftstoffdetergentien. Diese Tetrahydrobenzoxazine werden gemäß den in (3) und (4) genannten Herstellverfahren als Gemische mit den entsprechenden offenkettigen Mannich-Addukten des zugrundeliegenden Phenols erhalten und auch so in den Ottokraftstoffen eingesetzt.

Im Jounal of Polymer Science, Part A, Polymer Chemistry, Vol. 31, Seite 1941-1958 (5) beschreiben D. Jamois, M. Tessier und E. Maréchal Herstellverfahren für Polyisobutenb-polyethylenamine, welche sich als Schlammdispergiermittel in Motorenölen eignen. Unter den von diesen Autoren hergestellten Verbindungen sind unter anderem auch zweikernige phenolische Verbindungen, die über die jeweiligen ortho-Positionen der beiden Phenolkerne durch eine -CH₂-N(C₁₂H₂₅)-CH₂-Brücke verknüpft sind.

Aus der US 6 323 270 B1 (6) sind Oligomere oder Polymere von Tetrahydrobenzoxazinen bekannt, welche am Stickstoffatom und/oder am Benzolkern Alkylgruppen mit 1 bis 10 Kohlenstoffatomen oder Arylgruppen, Alkylarylgruppen oder Arylalkylgruppen mit jeweils 6 bis 20 Kohlenstoffatomen als Substituenten tragen können. Die genaue chemische Struktur solcher Tetrahydrobenzoxazin-Oligomerer oder -polymerer wird nicht angegeben. Diese Tetrahydrobenzoxazin-Oligomere oder -polymere eignen sich zur Herstellung von Bremsbelägen für Flugzeuge, keramischen Artikeln, Verpackungsmaterialien, Beschichtungen, Klebstoffe und Kompositmaterialien.

In der JP 2003-255 487 A (7) werden polymere mehrkernige phenolische Verbindungen offenbart, welche über die ortho-Positionen der Phenolkerne jeweils durch -CH₂-N(C₁-C₂₅-Alkyl)-CH₂-Brücken oder -CH₂-N(Phenyl)-CH₂-Brücken verknüpft sind. Auch die Phenolkerne selbst können durch C₁-C₂₅-Alkylreste substituiert sein. Die Anzahl der Phenolkerne in den Polymermolekülen wird mit 2 bis 1002 angegeben. Als konkrete Beispiele für die auftretenden C₁-C₂₅-Alkylreste werden Methyl, Ethyl, Butyl, Octyl und Dodecyl genannt. Substituenten, die eine oligomere oder polymere Verteilung aufweisen, werden nicht genannt. Diese polymeren mehrkernigen phenolischen Verbindungen werden als photothermographisch bilderzeugendes Material empfohlen.

Es besteht für den Mineralölprodukte- und Kraftstoff-Bereich ein Bedarf an Dispergatoren zur Verhinderung von Ablagerungen im Kraftstoffkreislauf und im Verbrennungssystem.

Es bestand daher die Aufgabe, Dispergatoren für den Mineralölprodukte- und Kraftstoffbereich mit einer verbesserten Wirkung bereitzustellen.

Demgemäß wurde die Verwendung von mehrkernigen phenolischen Verbindungen mit bis zu 20 Benzolkernen pro Molekül, welche durch Umsetzung eines Tetrahydrobenzoxazins der allgemeinen Formel I 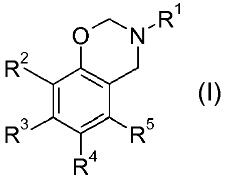
in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und

in der die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit einem oder mehreren der gleichen oder verschiedener Phenole der allgemeinen Formel II 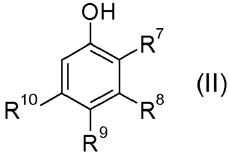 in der die Substituenten R⁷, R⁸, R⁹ und R¹⁰ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
und/oder mit einem oder mehreren der gleichen oder verschiedener Tetrahydrobenzoxazine der allgemeinen Formel I,
wobei der Substituent R⁴ auch für einen Rest der Formel Z und der Substituent R⁹ auch für einen Rest der Formel Z' stehen kann 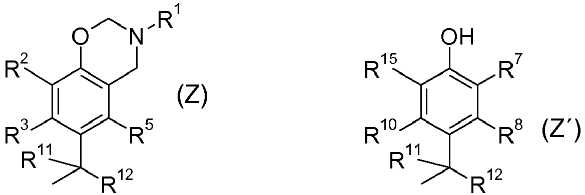
in denen die Substituenten R¹, R², R³, R⁵, R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR¹³-CH₂- auch einen zweiten Tetrahydrooxazin-Ring oder die Substituenten R² und R³ und R⁴ und R⁵ mit den an den Benzolkern angebundenen Teilstrukturen -O-CH₂-NR¹³-CH₂- und -O-CH₂-NR¹⁴-CH₂-auch einen zweiten und einen dritten Tetrahydrooxazin-Ring ausbilden können, wobei R¹³ und R¹⁴ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R⁷, R⁸, R⁹, R¹⁰, R¹³ oder R¹⁴ 13 bis 3000 Kohlenstoffatome aufweist und die übrigen Substituenten aus der Gruppe R¹, R², R³, R⁴, R⁵, R⁷, R⁸, R⁹, R¹⁰, R¹³ oder R¹⁴, wenn sie für Hydrocarbylreste stehen, jeweils 1 bis 20 Kohlenstoffatome aufweisen,
erhältlich sind, als Dispergatoren zur Verhinderung von Ablagerungen im Kraftstoffsystem und/oder Verbrennungssystem von Turbinen gefunden.

Die strukturelle Besonderheit der erfindungsgemäß zu verwendenden mehrkernigen phenolischen Verbindungen ist, dass sie mindestens einen längerkettigen Hydrocarbylrest mit 13 bis 3000 Kohlenstoffatomen als einen der Substituenten R¹, R², R³, R⁴, R⁵, R⁷, R⁸, R⁹, R¹⁰, R¹³ oder R¹⁴, welche aus den eingesetzten Tetrahydrobenzoxazinen I oder den Phenolen II stammen, enthalten. In einer bevorzugten Ausführungsform ist dieser längerkettige Hydrocarbylrest mit 13 bis 3000 Kohlenstoffatomen ein Polyisobutenylrest. Der genannte längerkettige Hydrocarbylrest kann in einer weiteren Ausführungsform auch einen C₁₆- bis C₂₀-Alkyl- oder -Alkenylrest bedeuten. Insbesondere sitzt dieser längerkettige Hydrocarbylrest, der vorzugsweise ein Polyisobutenylrest ist, an einem Oxazinring oder an einem Benzolkern in ortho- oder vorzugsweise in paraStellung zur phenolischen Hydroxylgruppe, d.h. er tritt als Substituent R¹ oder R² oder R⁴ oder R⁷ oder R⁹ oder R¹³ oder R¹⁴ auf. Dieser längerkettige Hydrocarbylrest, der vorzugsweise ein Polyisobutenylrest ist, umfasst vorzugsweise 21 bis 3000 oder vorzugsweise 21 bis 1000, insbesondere 26 bis 3000 oder insbesondere 26 bis 500, vor allem 30 bis 3000 oder vor allem 30 bis 250 Kohlenstoffatome. Im Falle von Polyisobutenylresten weisen diese zahlenmittlere Molekulargewichte Mₙ von 183 bis 42.000, vorzugsweise 500 bis 15.000, insbesondere 700 bis 7000, vor allem 900 bis 3000, ganz besonders bevorzugt 900 bis 1100 auf.

Als C₁₆- bis C₂₀-Alkyl- oder -Alkenylreste eignen sich zweckmäßigerweise die Reste von entsprechenden gesättigten oder ungesättigten Fettalkoholen mit 16 bis 20 Kohlenstoffatomen. Insbesondere sind hier n-Hexadecyl (Palmityl), n-Octadecyl (Stearyl), n-Eicosyl, Oleyl, Linolyl und Linolenyl zu nennen, welche gemäß ihrem natürlichen Vorkommen meist als technische Gemische untereinander auftreten.

Der genannte längerkettige Hydrocarbylrest mit 13 bis 3000 Kohlenstoffatomen kann in den mehrkernigen phenolischen Verbindungen auch mehrfach, beispielsweise zweifach oder dreifach, vertreten sein. In einer bevorzugten Ausführungsform treten ein oder zwei Polyisobutenyl-Reste mit einem jeweiligen zahlenmittleren Molekulargewicht Mₙ von 183 bis 42.000 im Molekül als Substituent R¹ und/oder R² und/oder R⁴ und/oder R⁷ und/oder R⁹ und/oder R¹³ und/oder R¹⁴ auf.

Die übrigen Substituenten aus der Gruppe R¹, R², R³, R⁴, R⁵, R⁷, R⁸, R⁹, R¹⁰, R¹³ oder R¹⁴, die nicht für Substituenten mit 13 bis 3000 Kohlenstoffatomen bzw. für Polyisobutenylreste mit einem zahlenmittleren Molekulargewichte Mₙ von 183 bis 42.000 stehen, bezeichnen unabhängig voneinander Wasserstoffatome, Hydroxylgruppen oder, wenn sie für Hydrocarbylreste stehen, meist kürzerkettige Hydrocarbylreste mit 1 bis 20, vorzugsweise 1 bis 12, vor allem 1 bis 8, ganz besonders bevorzugt lineare oder verzweigte C₁- bis C₄-Alkylreste. Typische Beispiele für die letzteren sind Methyl, Ethyl, n-Propyl, iso-Propyl, n-Butyl, 2-Butyl, sec.-Butyl und tert.-Butyl. Methylreste und tert.-Butylreste werden hierbei ganz besonders bevorzugt.

Bevorzugt erfindungsgemäß einzusetzende mehrkernige phenolische Verbindungen Tetrahydrobenzoxazine I sind auch solche, bei denen die aus den eingesetzten Tetrahydrobenzoxazinen I bzw. Phenolen II stammenden Substituenten R² und/oder R⁴ und/oder R⁷ und/oder R⁹, wenn sie für kürzerkettige Hydrocarbylreste stehen, lineare oder verzweigte C₁- bis C₄-Alkylreste, insbesondere Methylreste und/oder tert.-Butyl-reste, bezeichnen. Derartige Substitutionsmuster kommen natürlich nur bei Tetrahydrobenzoxazine I mit insgesamt einem oder zwei Tetrahydrooxazin-Ringsystemen in Betracht.

Im Rest der Formel Z bzw. Z' bezeichnen die Substituenten R¹¹ und R¹² vorzugsweise Wasserstoff und/oder lineare oder verzweigte C₁- bis C₄-Alkylreste, insbesondere Methylreste. Die Verbindungen I und II mit einem Rest Z bzw. Z', bei dem R¹¹ = R¹² = Methyl ist, leiten sich von Bisphenol A [2,2-Bis-(4-hydroxyphenyl)propan] ab. Herstellungsbedingt können Verbindungen I mit einem Rest Z und Verbindungen I mit dem entsprechenden Rest Z' auch als Mischungen vorliegen.

Unter Hydrocarbylresten mit 1 bis 3000 bzw. 13 bis 3000 Kohlenstoffatomen für die Substituenten R¹, R², R³, R⁴, R⁵, R⁷, R⁸, R⁹, R¹⁰, R¹³ und R¹⁴ sollen hier reine Kohlenwasserstoffreste jeglicher Struktur verstanden werden, die definitionsgemäß auch noch durch ein oder mehrere Heteroatome aus der Gruppe O und S und/oder durch eine oder mehrere Gruppierungen NR⁶ unterbrochen sein können. Ein typischer durch eine NR⁶-Gruppierung unterbrochener Hydrocarbylrest leitet sich von 3-(Dimethylamino)-propylamin ab. Insbesondere sind Hydrocarbylreste Alkyl-, Alkenyl-, Cycloalkyl-, Aryl-, Alkylaryl-, Alkenylaryl- oder Arylalkyl-Reste.

Bei Unterbrechungen des Hydrocarbylrestes durch Gruppierungen NR⁶ sind auch solche Reste gemeint, bei denen am Ende die Gruppierungen NR⁶ formal in eine C-H-Bindung insertiert ist, also beispielsweise Substituenten R¹, R², R³, R⁴, R⁵, R⁷, R⁸, R⁹, R¹⁰, R¹³ oder R¹⁴ mit einer NH₂-Endgruppe. Derartige Hydrocarbylreste leiten sich z.B. von Polyaminen wie Ethylendiamin, Diethylentriamin, Triethylentetramin, Tetraethylenpentamin, etc. ab, bei denen eines der endständigen Stickstoffatome das N-Atom im Oxazin-Ring darstellt.

Der Ausdruck "Alkyl" umfasst geradkettige und verzweigte Alkylgruppen. Beispiele für Alkylgruppen sind neben den bereits oben genannten Methyl-, Ethyl-, n-Propyl-, iso-Propyl-, n-Butyl-, 2-Butyl-, sec.-Butyl- und tert.-Butyl-Resten insbesondere auch n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethyl-butyl, 2,2-Dimethylbutyl, 3,3-Dimethylbutyl, 1,1,2-Trimethylpropyl, 1,2,2-Trimethyl-propyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Dodecyl, n-Tridecyl, iso-Tridecyl, n-Tetradecyl (Myristyl), n-Hexadecyl (Palmityl), n-Octadecyl (Stearyl), und n-Eicosyl.

Beispiele für Alkenylreste sind Vinyl, 1-Propenyl, 2-Propenyl, Oleyl, Linolyl und Linolenyl.

Beispiele für Cylcoalkylreste sind C₅- bis C₇-Cycloalkylgruppen wie Cyclopentyl, Cyclohexyl und Cycloheptyl, welche noch durch Alkylgruppen, beispielsweise Methylreste, substituiert sein können.

Der Ausdruck "Aryl" umfasst einkernige, zweikernige, dreikernige und höherkernige aromatische Kohlenwasserstoffreste. Diese Arylreste können im Falle einer Substitution durch die beispielsweise vorgenannten Alkyl- und/oder Alkenylreste zu Alkylaryl- bzw. Alkenylarylresten noch 1, 2, 3, 4 oder 5, vorzugsweise 1, 2 oder 3 Substituenten tragen. Typische Beispiele sind Phenyl, Tolyl, Xylyl, Mesityl, Naphthyl, Fluorenyl, Anthracenyl, Phenanthrenyl, Naphthacenyl und Styryl. Ein typisches Beispiel für einen Arylalkylrest ist Benzyl.

Ist der längerkettige Hydrocarbylrest mit 13 bis 3000 Kohlenstoffatomen ein Polyisobutenylrest, kann er auf prinzipiell jedem gängigen und kommerziell erhältlichen Polyisobuten basieren, welches in geeigneter Weise in die Synthese der Tetrahydrobenzoxazine I eingebracht wird. Ein solches Polyisobuten besitzt ein zahlenmittleres Molekulargewicht Mₙ von mindestens 183. Bevorzugt sind Polyisobutene mit einem zahlenmittleren Molekulargewicht Mₙ im Bereich von 183 bis 42.000, besonders bevorzugt von 500 bis 15.000, vor allem von 700 bis 7000, insbesondere von 900 bis 3000 und ganz besonders bevorzugt von 900 bis 1100. Unter den Begriff "Polyisobuten" fallen im Sinne der vorliegenden Erfindung auch oligomere Isobutene wie dimeres, trimeres, tetrameres, pentameres, hexameres und heptameres Isobuten.

Vorzugsweise leiten sich die in den erfindungsgemäß verwendeten mehrkernigen phenolischen Verbindungen eingebauten Polyisobutenylreste von sogenanntem "reaktiven" Polyisobuten ab. Reaktive Polyisobutene unterscheiden sich von den konventionellen Polyisobutenen durch den Gehalt an terminal angeordneten Doppelbindungen, d.h. an Vinyliden-Doppelbindungen [-CH-C(CH₃)=CH₂] (α-Olefin) oder Vinyl-Doppelbindungen [-CH=C(CH₃)₂] (β-Olefin). So enthalten reaktive Polyisobutene wenigstens 50 Mol-% terminal angeordnete Doppelbindungen, bezogen auf die Gesamtanzahl an Polyisobuten-Makromolekülen. Besonders bevorzugt sind Polyisobutene mit wenigstens 60 Mol-% und insbesondere mit wenigstens 80 Mol-% terminal angeordnete Doppelbindungen, bezogen auf die Gesamtanzahl an Polyisobuten-Makromolekülen. Außerdem weisen die im Wesentlichen homopolymere Polyisobutenylreste einheitliche Polymergerüste auf. Darunter werden im Rahmen der vorliegenden Erfindung solche Polyisobuten-Systeme verstanden, die zu wenigstens 85 Gew.-%, vorzugsweise zu wenigstens 90 Gew.-%, besonders bevorzugt zu wenigstens 95 Gew.-% und insbesondere zu wengistens 99 Gew.-% aus Isobuteneinheiten der Wiederholungseinheit [-CH₂C(CH₃)₂-] aufgebaut sind.
Ein weiteres bevorzugtes Merkmal der Polyisobutene, die den erfindungsgemäß verwendeten mehrkernigen phenolischen Verbindungen zugrunde liegen können, ist, dass sie zu wenigstens 15 Gew.-%, insbesondere zu wenigstens 50 Gew.-%, vor allem zu wenigstens 80 Gew.-% mit einer tert.-Butylgruppe [-CH₂C(CH₃)₃] terminiert sind.

Weiterhin weisen die als Basis für die als Ausgangsmaterial für die erfindungsgemäß verwendeten mehrkernigen phenolischen Verbindungen verwendeten Tetrahydrobenzoxazine I bzw. Phenole II vorzugsweise dienenden Polyisobutene vorzugsweise einen Polydispersitätsindex (PDI) von 1,05 bis 10, vorzugsweise von 1,05 bis 3,0, insbesondere von 1,05 bis 2,0 auf. Unter Polydispersität versteht man den Quotienten aus gewichtsmittlerem Molekulargewicht M_{w} und zahlenmittlerem Molekulargewicht Mₙ (PDI = M_{w}/Mₙ). In einer bevorzugten Ausführungsform beträgt der gemittelte Polydispersitätsindex PDI für die Polyisobutenyl-Reste im den mehrkernigen phenolischen Verbindungen höchstens das 5-fache, vorzugsweise höchstens das 3-fache, insbesondere höchstens das 2-fache, vor allem höchstens das 1,5-fache, des gemittelten Polydispersitätsindex PDI für die Polyisobutenyl-Reste in den zugrundeliegenden Tetrahydrobenzoxazinen I und/oder Phenolen II.

Unter als Basis für die Tetrahydrobenzoxazine I bzw. Phenole II vorzugsweise dienenden Polyisobutenen werden im Sinne der vorliegenden Erfindungen auch alle durch kationische Polymerisation erhältlichen Polymerisate verstanden, die vorzugsweise wenigstens 60 Gew.-% Isobuten, besonders bevorzugt wenigstens 80 Gew.-%, vor allem wenigstens 90 Gew.-% und insbesondere wenigstens 95 Gew.-% Isobuten einpolymerisiert enthalten. Daneben können die Polyisobutene weitere Buten-Isomere wie 1- oder 2-Buten sowie davon verschiedene olefinisch ungesättigte Monomere, die mit Isobuten unter kationischen Polymerisationsbedingungen copolymerisierbar sind, einpolymerisiert enthalten.

Als Isobuten-Einsatzstoffe für die Herstellung von Polyisobutenen, die als Basis für die Tetrahydrobenzoxazine I bzw. Phenole II dienen können, eignen sich dementsprechend sowohl Isobuten selbst als auch isobutenhaltige C₄-Kohlenwasserstoffströme, beispielsweise C₄-Raffinate, C₄-Schnitte aus der Isobuten-Dehydrierung, C₄-Schnitte aus Steamcrackern, FCC-Crackern (FCC: Fluid Catalyzed Cracking), sofern sie weitgehend von darin enthaltenem 1,3-Butadien befreit sind. Besonders geeignete C₄-Kohlenwasserstoffströme enthalten in der Regel weniger als 500 ppm, vorzugsweise weniger als 200 ppm Butadien. Bei Einsatz von C₄-Schnitten als Einsatzmaterial übernehmen die von Isobuten verschiedenen Kohlenwasserstoffe die Rolle eines inerten Lösungsmittels.

Als mit Isobuten copolymerisierbare Monomere kommen Vinylaromaten wie Styrol und a-Methylstyrol, C₁-C₄-Alkylstyrole wie 2-, 3- und 4-Methylstyrol, sowie 4-tert.-Butylstyrol, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht.

Typische Polyisobutene, die als Basis für die Tetrahydrobenzoxazine I bzw. Phenole II dienen können, sind beispielsweise die Glissopal®-Marken der BASF Aktiengesellschaft, z. B. Glissopal 550, Glissopal 1000 und Glissopal 2300, sowie die Oppanol®-Marken der BASF Aktiengesellschaft, z.B. Oppanol B10, B12 und B15.

Neben Polyisobutenylresten können als längerkettige Hydrocarbylreste auch solche auftreten, die sich von Oligomeren oder Polymeren von C₂- bis C₁₂-Olefinen ableiten und im Mittel 13 bis 3000 Kohlenstoffatome aufweisen. Derartige meist polydisperse Hydrocarbylreste mit polymerer Verteilung sind beispielsweise solche, die sich von Ethylen, Propylen, Buten, Styrol, Methylstyrol, Hexen-1, Octen-1, Decen-1 oder Dodecen-1 ableiten. Sie können Homo- oder Copolymer-Reste sein. Ihr zahlenmittleres Molekulargewicht Mₙ beträgt mindestens 183, ihr Polydispersitätsindex PDI üblicherweise 1,05 bis 10. Bei niedermolekularen Resten mit Mₙ von 183 bis ca. 500 können sie auch monodispers vorliegen.

In einer bevorzugten Ausführungsform weisen die erfindungsgemäß verwendeten mehrkernigen phenolischen Verbindungen ein mittleres Molekulargewicht Mₙ von 411 bis 25.000 auf. So steht beispielsweise das Molekulargewicht Mₙ von 411 für die kleinsten Vertreter der mehrkernigen phenolischen Verbindungen im Rahmen der vorliegenden Erfindung, nämlich Bis-(ortho- oder para-hydroxybenzyl)-tridecylamin. Besonders bevorzugte Bereiche für Mₙ sind 523 bis 25.000 oder 523 bis 17.000, insbesondere 593 bis 25.000 oder 593 bis 10.000, vor allem 649 bis 25.000 oder 649 bis 5000.

Beispiele für im Sinne der vorliegenden Erfindung typische mehrkernige phenolische Verbindungen sind die folgenden, wobei "PIB" einen von einem hochreaktiven Polyisobuten (Mₙ 1000) abgeleiteten Polyisobutenylrest bezeichnet: 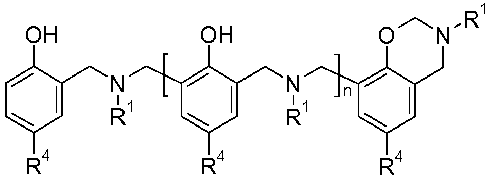
(IIIa) n = 0, R¹ = PIB, R⁴ = H
(IIIb) n = 0, R¹ = Methyl, R⁴ = PIB
(IIIc) n = 0, R¹ = PIB, R⁴ = tert.-Butyl

(IIId) n = 1, R¹ = PIB, R⁴ = H
(IIIe) n = 1, R¹ = Methyl, R⁴ = PIB
(IIIf) n = 1, R¹ = PIB, R⁴ = tert.-Butyl

(IIIg) n = 2, R¹ = PIB, R⁴ = H
(IIIh) n = 2, R¹ = Methyl, R⁴ = PIB
(IIIi) n = 2, R¹ = PIB, R⁴ = tert.-Butyl

(IIIj) n = 3, R¹ = PIB, R⁴ = H
(IIIk) n = 3, R¹ = Methyl, R⁴ = PIB
(IIIl) n = 3, R¹ = PIB, R⁴ = tert.-Butyl

(IIIm) n = 4, R¹ = PIB, R⁴ = H
(IIIn) n = 4, R¹ = Methyl, R⁴ = PIB
(IIIo) n = 4, R¹ = PIB, R⁴ = tert.-Butyl

(IIIp) n = 5, R¹ = PIB, R⁴ = H
(IIIq) n = 5, R¹ = Methyl, R⁴ = PIB
(IIIr) n = 5, R¹ = PIB, R⁴ = tert.-Butyl

(IIIs) n = 6, R¹ = PIB, R⁴ = H
(IIIt) n = 6, R¹ = Methyl, R⁴ = PIB
(IIIu) n = 6, R¹ = PIB, R⁴ = tert.-Butyl

(IIIv) n = 1, R¹ = Methyl,
   1 Rest R⁴ = PIB, 2 Reste R⁴ = tert.Butyl
(IIIw) n = 8, R¹ = Methyl,
   1 Rest R⁴ = PIB, 9 Reste R⁴ = tert.Butyl

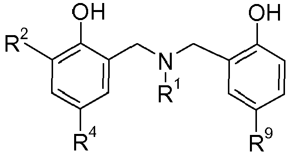

(IVa) R¹ = Methyl, R² = H, R⁴ = tert.-Butyl, R⁹ = PIB
(IVb) R¹ = Methyl, R² = R⁴ = tert.-Butyl, R⁹ = PIB
(IVc) R¹ = PIB, R² = R⁴ = tert.-Butyl, R⁹ = H
(IVd) R¹ = PIB, R² = R⁴ = R⁹ = H
(IVe) R¹ = PIB, R² = R⁴ = H, R⁹ = tert.-Butyl
(IVf) R¹ = PIB, R² = H, R⁴ = tert.-Butyl, R⁹ = PIB
(IVg) R¹ = PIB, R² = R⁴ = tert.-Butyl, R⁹ = PIB
(IVh) R¹ = PIB, R² = R⁴ = H, R⁹ =

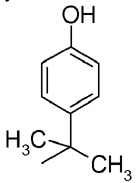

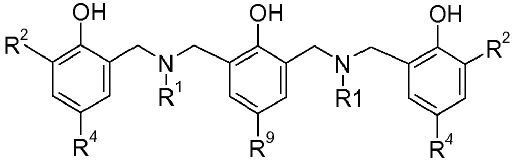

(Va) R¹ = Methyl, R² = R⁴ = H, R⁹ = PIB
(Vb) R¹ = Methyl, R² = R⁴ = tert.-Butyl, R⁹ = PIB
(Vc) R¹ = Methyl, R² = tert.-Butyl, R⁴ = Methyl, R⁹ = PIB
(Vd) R¹ = R² = Methyl, R⁴ = tert.-Butyl, R⁹ = PIB
(Ve) R¹ = 3-(Dimethylamino)propyl, R² = R⁴ = tert.-Butyl, R⁹ = PIB
(Vf) R¹ = PIB, R² = R⁴ = R⁹ = H
(Vg) R¹ = PIB, R² = R⁴ = H, R⁹ = tert.-Butyl
(Vh) R¹ = PIB, R² = R⁴ = tert.-Butyl, R⁹ = H
(Vi) R¹ = PIB, R² = H, R⁴ = R⁹ = tert.-Butyl
(Vj) R¹ = PIB, R² = R⁴ = R⁹ = tert.-Butyl
(Vk) R¹ = PIB, R² = R⁴ = H, R⁹ = PIB
(Vm) R¹ = PIB, R² = R⁴ = H, R⁹ = 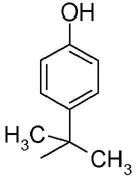
(Vn) R¹ = 3-(Dimethylamino)propyl, R² = tert.-Butyl, R⁴ = Methyl,
   R⁹ = PIB

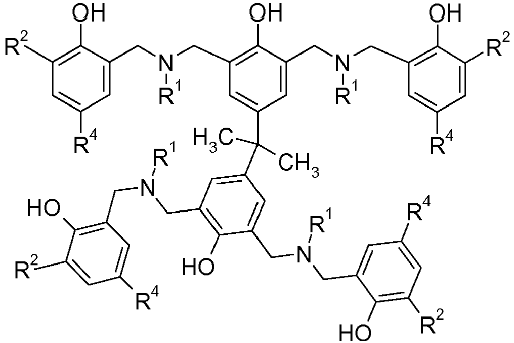

(VIa) R¹ = Methyl, R² = tert.-Butyl, 3 Reste R⁴ = tert.-Butyl, 1 Rest R⁴ = PIB
(VIb) R¹ = Methyl, R² = tert.-Butyl, 3 Reste R⁴ = Methyl, 1 Rest R⁴ = PIB
(VIc) R¹ = Methyl, 3 Reste R² = tert.-Butyl, 1 Rest R² = H,
   3 Reste R⁴ = tert.-Butyl, 1 Rest R⁴ (am Benzolkern mit R² = H) = PIB

Die beschriebenen mehrkernigen phenolischen Verbindungen eignen sich als Stabilisatoren in Turbinenkraftstoffen (jet fuels). Hierunter ist auch ihre Wirkungsweise als Antioxidantien im herkömmlichen Sinne zu verstehen. Insbesondere dienen sie über ihre Wirkungsweise als Stabilisatoren zur Verbesserung der thermischen Stabilität von Turbinenkraftstoffen. Sie verhindern gemäß der vorliegenden Erfindung über ihre Wirkungsweise als Stabilisatoren, d.h. in ihrer Eigenschaft als Dispergatoren, Ablagerungen im Kraftstoffsystem und/oder Verbrennungssystem von Turbinen. Turbinenkraftstoff werden vor allem zum Betreiben von Flugzeugturbinen eingesetzt.

Eine Turbinenkraftstoffzusammensetzung, die einen Turbinenkraftstoff (jet fuel) und wenigstens eine der beschriebenen mehrkernigen phenolischen Verbindungen enthält, enthält eine Hauptmenge eines flüssigen Turbinenkraftstoffs, wobei es sich beispielsweise um einen in der zivilen oder militärischen Luftfahrt üblichen Turbinenkraftstoff handelt. Dazu zählen beispielsweise Kraftstoffe der Bezeichnung Jet Fuel A, Jet Fuel A-1, Jet Fuel B, Jet Fuel JP-4, JP-5, JP-7, JP-8 und JP-8+100. Jet A und Jet A-1 sind kommerziell erhältliche Turbinenkraftstoffspezifikationen auf Kerosinbasis. Die zugehörigen Normen sind ASTM D 1655 sowie DEF STAN 91-91. Jet B ist ein weiter geschnittener Kraftstoff auf Basis von Naphtha- und Kerosinfraktionen. JP-4 ist äquivalent zu Jet B. JP-5, JP-7, JP-8 und JP-8+100 sind militärische Turbinenkraftstoffe, wie sie beispielsweise von der Marine und Luftwaffe eingesetzt werden. Zum Teil bezeichnen diese Normen Formulierungen, die bereits weitere Additive, wie Korrosionsinhibitoren, Vereisungsinhibitoren, statische Dissipatoren, etc. enthalten.

Die beschriebenen mehrkernigen phenolischen Verbindungen können dem Turbinenkraftstoff oder der Turbinenkraftstoffzusammensetzung einzeln, als Gemische und gegebenenfalls in Kombination mit weiteren an sich bekannten Zusatzstoffen zugegeben werden.

Geeignete Zusatzstoffe, die in der erfindungsgemäßen Turbinenkraftstoffzusammensetzung enthalten sein können, umfassen üblicherweise Detergenzien, Korrosionsinhibitoren, weitere Antioxidantien wie sterisch gehinderte tert.-Butylphenole, N-Butylphenylendiamine oder N,N'-Diphenylamin und Derivate hiervon, Metalldesaktivatoren wie N,N'-Disalicyliden-1,2-diaminopropan, Lösungsvermittler, Antistatika wie Stadis 450, Biocide, Anti-Icing-Mittel wie Diethylenglykolmethylether, sowie Mischungen der genannten Zusatzstoffe.

Bevorzugte Zusatzstoffe sind die nachfolgend aufgeführten speziellen Verbindungsklassen (A), (B) und (C):
Bevorzugte Zusatzstoffe (A) sind von Bernsteinsäureanhydrid abgeleitete Verbindungen mit langkettigen Kohlenwasserstoffresten mit in der Regel 15 bis 700, vor allem 30 bis 200 Kohlenstoffatomen. Diese Verbindungen können weitere funktionelle Gruppen aufweisen, die vorzugsweise ausgewählt sind unter Hydroxy-, Amino-, Amido- und/oder Imidogruppen. Bevorzugte Additive sind die entsprechenden Derivate von Polyalkenylbernsteinsäureanhydrid, welche z. B. durch Umsetzung von Polyalkenen mit Maleinsäureanhydrid auf thermischem Weg oder über die chlorierten Kohlenwasserstoffe erhältlich sind. Das zahlenmittlere Molekulargewicht der langkettigen Kohlenwasserstoffreste liegt vorzugsweise in einem Bereich von etwa 200 bis 10.000, besonders bevorzugt 400 bis 5000, insbesondere 600 bis 3000 und speziell 650 bis 2000. Vorzugsweise leiten sich diese langkettigen Kohlenwasserstoffreste von konventionellen und insbesondere von den zuvor genannten reaktiven Polyisobutenen ab. Von besonderem Interesse als Zusatzstoffe (A) sind die Derivate von Polyalkenylbernsteinsäureanhydriden mit Ammoniak, Monoaminen, Polyaminen, Monoalkoholen und Polyolen. Zur Derivatisierung bevorzugte Polyamine umfassen Ethylendiamin, Diethylentriamin, Triethylentetramin, Tetraethylenpentamin, Propylendiamin, etc. Geeignete Alkohole umfassen einwertige Alkohole, wie Ethanol, Allylalkohol, Dodecanol und Benzylalkohol, mehrwertige Alkohole, wie Ethylenglykol, Diethylenglykol, Propylenglykol, 1,2-Butandiol, Neopentylglykol, Glycerin, Trimethylolpropan, Erythrit, Pentaerythrit, Mannitol und Sorbitol.

Als Zusatzstoffe geeignete Bernsteinsäureanhydrid-Derivate (A) sind beispielsweise in der US 3 522 179, US 4 234 435, US 4 849 572, US 4 904 401, US 5 569 644 und US 6 165 235 beschrieben, worauf hier in vollem Umfang Bezug genommen wird.

Bevorzugte Zusatzstoffe (B) sind Polyalkenylthiophosphonatester. Der Polyalkenylrest diese Ester weist vorzugsweise ein zahlenmittleres Molekulargewicht im Bereich von etwa 300 bis 5000, besonders bevorzugt 400 bis 2000 und insbesondere 500 bis 1500 auf. Der Polyalkenylrest leitet sich vorzugsweise von Polyolefinen ab, wie sie zuvor bei der Komponente (A) als langkettiger Kohlenwasserstoffrest beschrieben wurden. Dabei handelt es sich speziell um Polyalkenylreste, die sich von konventionellen oder reaktiven Polyisobutenen ableiten. Geeignete Verfahren zur Herstellung geeigneter Polyalkenylthiophosphonatester durch Umsetzung eines Polyolefins mit einem Thiophosphorylierungsmittel sind z. B. in der US 5 725 611 beschrieben, worauf hier Bezug genommen wird.

Bevorzugte Zusatzstoffe (C) sind Mannich-Addukte. Derartige Addukte werden prinzipiell durch Mannich-Umsetzung von aromatischen Hydroxylverbindungen, insbesondere Phenol und Phenolderivaten, mit Aldehyden und Mono- oder Polyaminen erhalten. Vorzugsweise handelt es sich um die Umsetzungsprodukte von Polyisobutensubstituierten Phenolen mit Formaldehyd und Mono- oder Polyaminen wie Ethylendiamin, Diethylentriamin, Triethylentetramin, Tetraethylenpentamin, Dimethylaminopropylamin, etc. Geeignete Mannich-Addukte und Verfahren zu ihrer Herstellung sind z. B. in der US 5 876 468, EP-A 831 141, EP-A 1 233 990 und EP-A 1 226 188 beschrieben, worauf hier in vollem Umfang Bezug genommen wird.

Die Turbinenkraftstoffzusammensetzung enthält die beschriebenen mehrkernigen phenolischen Verbindungen in einer Menge von üblicherweise 0,0001 bis 1 Gew.-%, vorzugsweise 0,001 bis 0,5 Gew.-%, insbesondere 0,01 bis 0,2 Gew.-% und vor allem 0,01 bis 0,1 Gew.-%, bezogen auf die Gesamtmenge der Turbinenkraftstoffzusammensetzung.

Die Zusatzstoffe (A) bis (C) sowie gegebenenfalls weitere der zuvor genannten Zusatzstoffe können üblicherweise jeweils in Mengen von jeweils 0,0001 bis 1 Gew.-%, vorzugsweise 0,001 bis 0,6 Gew.-% und insbesondere 0,0015 bis 0,4 Gew.-%, bezogen auf die Gesamtmenge der Turbinenkraftstoffzusammensetzung, eingesetzt werden.
Ein Additivkonzentrat für Turbinenkraftstoffe (jet fuels), welches wenigstens eine der beschriebenen mehrkernigen phenolischen Verbindungen sowie gegebenenfalls wenigstens ein Verdünnungsmittel sowie gegebenenfalls mindestens einen weiteren Zusatzstoff enthält, enthält, wie dann auch die Turbinenkraftstoffzusammensetzung, vorzugsweise neben dem Additivkonzentrat einen oder mehrere Zusatzstoffe aus der Gruppe (A), (B) und (C), insbesondere auch Mischungen hieraus wie (A) + (B), (A) + (C), (B) + (C) und (A) + (B) + (C).

Geeignete Verdünnungsmittel sind beispielsweise bei der Erdölverarbeitung anfallende Fraktionen wie Kerosin, Naphtha oder mineralische Grundöle. Geeignet sind darüber hinaus aromatische und aliphatische Kohlenwasserstoffe wie Solvent Naphtha schwer, Solvesso® oder Shellsol® sowie Gemische dieser Lösungs- und Verdünnungsmittel.

Die Erfindung wir anhand der folgenden, nicht einschränkenden Beispiele näher erläutert.

### Herstellungsbeispiele

### Beispiel 1:

### Verbindung der Formel Vb durch Umsetzung von 4-Polyisobutenylphenol mit 3-Methyl-6,8-di-tert.-butyltetrahydrobenzoxazin

In einem 500 ml-Vierhalskolben wurden 120 g 4-Polyisobutenylphenol (auf Basis des im Handel erhältlichen hochreaktiven Polyisobutens Glissopal® 1000 mit einem Mₙ von 1000) bei Raumtemperatur in 100 ml Toluol vorgelegt. 52 g 3-Methyl-6,8-di-tert.-butyltetrahydrobenzoxazin wurden rasch zugesetzt. Der Kolbeninhalt wurde bis zum Rückfluß erwärmt und 2 Stunden unter Rückfluß gerührt. Nach Abkühlung auf Raumtemperatur wurde der Kolbeninhalt mit Methanol gewaschen und die Toluolphase wurde bei 150°C und 5 mbar eingedampft. Man erhielt 113 g Produkt in Form eines hellen, klaren, zähen Öls.
1H-NMR (400 MHz, 16 Scans, CDCl₃):
Die Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-;) sowie das Signal für ein ortho-Proton des 4-Polyisobutenylphenols (δ = 6,70 ppm) sind vollständig verschwunden;
Vorhanden sind im Bereich δ = 3,8-3,5 ppm die Benzylprotonen, im Bereich δ = 2,6-2,0 ppm die Protonen der N-Methylgruppe und im Bereich δ = 6,9-7,2 ppm die Aromatenprotonen.

### Beispiel 2:

### Verbindung der Formel Ve durch Umsetzung von 4-Polyisobutenylphenol mit 3-(Dimethylamino)-propyl-6,8-di-tert.-butyltetrahydrobenzoxazin

In einem 1000 ml-Vierhalskolben wurden 170 g 4-Polyisobutenylphenol (auf Basis des im Handel erhältlichen hochreaktiven Polyisobutens Glissopal® 1000 mit einem Mₙ von 1000) bei Raumtemperatur in 100 ml Toluol vorgelegt. 100 g 3-(Dimethylamino)propyl-6,8-di-tert.-butyltetrahydrobenzoxazin wurden rasch zugesetzt. Der Kolbeninhalt wurde bis zum Rückfluß erwärmt und 2 Stunden unter Rückfluß gerührt. Nach Abkühlung auf Raumtemperatur wurde der Kolbeninhalt mit Methanol gewaschen und die Toluolphase wurde bei 90°C und 5 mbar eingedampft. Man erhielt 225 g Produkt in Form eines hellen, klaren, zähen Öls.
1H-NMR (400 MHz, 16 Scans, CDCl₃):
5-10% Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des 4-Polyisobutenylphenols (δ = 6,70 ppm) zeigen 90 bis 95 % Umsatz;
Vorhanden sind im Bereich δ = 3,8-3,4 ppm die Benzylprotonen, im Bereich δ = 2,6-2,0 ppm die Protonen der N-Methyl- und N-Methylengruppen und im Bereich δ = 7,0-7,3 ppm die Aromatenprotonen.

### Beispiel 3:

### Verbindung der Formel Vc durch Umsetzung von 4-Polyisobutenylphenol mit 3,6-Dimethyl-8-tert.-butyltetrahydrobenzoxazin

In einem 1000 ml-Vierhalskolben wurden 240 g 4-Polyisobutenylphenol (auf Basis des im Handel erhältlichen hochreaktiven Polyisobutens Glissopal® 1000 mit einem Mₙ von 1000) bei Raumtemperatur in 200 ml Toluol vorgelegt. 88 g 3,6-Dimethyl-8-tert.-butyl-tetrahydrobenzoxazin wurden rasch zugesetzt. Der Kolbeninhalt wurde bis zum Rückfluß erwärmt und 2 Stunden unter Rückfluß gerührt. Die Toluolphase wurde bei 150°C und 5 mbar eingedampft. Man erhielt 313 g Produkt in Form eines hellen, klaren, zähen Öls.
1H-NMR (400 MHz, 16 Scans, CDCl₃):
Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des 4-Polyisobutenylphenols (δ = 6,70 ppm) zeigen 80 % Umsatz;
Vorhanden sind im Bereich δ = 3,8-3,4 ppm die Benzylprotonen, im Bereich δ = 2,6-2,0 ppm die Protonen der N-Methylgruppe und im Bereich δ = 7,0-7,3 ppm die Aromatenprotonen.

### Beispiel 4:

### Verbindung der Formel IIIv durch Umsetzung von 3-Methyl-6-polyisobutenyltetrahydrobenzoxazin mit 3-Methyl-6-tert.-butyltetrahydrobenzoxazin

In einer 500 ml-Verdampferbirne wurden 100 g 3-Methyl-6-polyisobutenyltetrahydrobenzoxazin (auf Basis des im Handel erhältlichen hochreaktiven Polyisobutens Glissopal® 1000 mit einem Mₙ von 1000) bei Raumtemperatur in 200 ml Toluol vorgelegt. 16,4 g 3-Methyl-6-tert.-butyltetrahydrobenzoxazin wurden rasch zugesetzt. Das Toluol wurde am Rotationsverdampfer bei 100°C / 5 mbar abdestilliert und es wurde 4 Stunden bei 160°C / 900 mbar gerührt. Man erhielt 110 g Produkt in Form eines viskosen, zähen Öls.
1H-NMR (400 MHz, 16 Scans, CDCl₃):
Bezogen auf das Integral der Methylprotonen (Benzoxazin: 2,45-2,65 ppm; 2-Hydroxybenzylamin: 2,05-2,30 ppm) sind zwei Phenoleinheiten mit einem Benzoxazin verknüpft.

### Beispiel 5:

### Verbindung der Formel IIIw durch Umsetzung von 3-Methyl-6-polyisobutenyltetrahydrobenzoxazin mit 3-Methyl-6-tert.-butyltetrahydrobenzoxazin

In einer 500 ml-Verdampferbirne wurden 50 g 3-Methyl-6-polyisobutenyltetrahydrobenzoxazin (auf Basis des im Handel erhältlichen hochreaktiven Polyisobutens Glissopal® 1000 mit einem Mₙ von 1000) bei Raumtemperatur in 200 ml Toluol vorgelegt. 41 g 3-Methyl-6-tert.-butyltetrahydrobenzoxazin wurden rasch zugesetzt. Das Toluol wurde am Rotationsverdampfer bei 100°C / 5 mbar abdestilliert und es wurde 4 Stunden bei 160°C / 900 mbar gerührt. Man erhielt 75 g Produkt in Form eines viskosen, zähen Öls.
1H-NMR (400 MHz, 16 Scans, CDCl₃):
Bezogen auf das Integral der Methylprotonen, (Benzoxazin: 2,45-2,65ppm; 2-Hydroxybenzylamin: 2,05-2,30 ppm) sind 9 Phenoleinheiten mit einem Benzoxazin verknüpft.

### Beispiel 6:

### Verbindung der Formel Vlc durch Umsetzung von 3-Methyl-6-polyisobute-nyl-tetrahydrobenzoxazin und 3-Methyl-6,8-di-tert.-butyltetrahydrobenzoxazin mit Bisphenol A

In einem 1000 ml-Vierhalskolben wurden 130 g 3-Methyl-6-polyisobutenyltetrahydrobenzoxazin (auf Basis des im Handel erhältlichen hochreaktiven Polyisobutens Glissopal® 1000 mit einem Mₙ von 1000) 78,5 g 3-Methyl-6,8-di-tert.-butyltetrahydrobenzoxazin, 22,8 g Bisphenol A und 200 ml Toluol 2 Stunden unter Rückfluß gerührt. Der Kolbeninhalt wurde bei 140°C und 5 mbar eingedampft. Man erhielt 194,5 g Produkt in Form eines hellen, klaren, zähen Öls.
1H-NMR (400 MHz, 16 Scans, CDCl₃):
5% Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des Bisphenol A (δ = 6,60 ppm) zeigen 95 % Umsatz;
Vorhanden sind im Bereich δ = 3,8-3,4 ppm die Benzylprotonen, im Bereich δ = 2,6-2,0 ppm die Protonen der N-Methylgruppen und im Bereich δ = 6,7-7,3 ppm die Aromatenprotonen.

### Beispiel 7: Verbindung der Formel Vn durch Umsetzung von 4-Polyisobutenylphenol mit 3-(Dimethylamino)-propyl-6-methyl-8-tert.-butyltetrahydrobenzoxazin

In einem 1000 ml-Vierhalskolben wurden 300 g 4-Polyisobutenylphenol (auf Basis des im Handel erhältlichen hochreaktiven Polyisobutens Glissopal® 1000 mit einem Mₙ von 1000) bei Raumtemperatur in 190 g Solvesso® 150 (Solvent Naphta Heavy) vorgelegt. 145 g 3-(Dimethylamino)-propyl-6-methyl-8-tert.-butyltetrahydrobenzoxazin wurden rasch zugesetzt. Der Kolbeninhalt wurde 4 Stunden bei 150°C gerührt. Man erhielt 630 g einer hellen, klaren Lösung des Produktes (70 Gew.-% Feststoffgehalt). Für die analytische Bestimmung wurde ein Probe bei 150°C und 3 mbar eingedampft.
1H-NMR (400 MHz, 16 Scans, CDCl₃):
Rest-Tetrahydrobenzoxazin-Signale (δ = 4,80 ppm, 2H, -CH₂-N(-CH₃)-CH₂-O-; δ = 3,91 ppm, 2H, -CH₂-N(-CH₃)-CH₂-O-) sowie ein geringes Restsignal für ein ortho-Proton des 4-Polyisobutenylphenols (δ = 6,70 ppm) zeigen 90 % Umsatz;
Vorhanden sind im Bereich δ = 3,8-3,4 ppm die Benzylprotonen, im Bereich δ = 2,6-2,0 ppm die Protonen der N-Methylgruppe und im Bereich δ = 6,7-7,3 ppm die Aromatenprotonen.

### Anwendungsbeispiele

### Beispiel 8: Überprüfung der thermischen Stabilität von Turbinenkraftstoff (jet fuel)

Es wurde ein Turbinenkraftstoff der Spezifikation Jet A-1 nach DEF STAN 91-91 bzw. ASTM D 1655 eingesetzt. Die Additivierung erfolgte mit jeweils 100 mg/l der Verbindungen aus den Herstellungsbeispielen 1 bis 6.

In einem Dreihals-Glaskolben, der mit Rührer, Rückflusskühler und Thermometer versehen war, wurden zunächst bei Raumtemperatur 5 I Luft innerhalb 1 h durch 150 ml des zu untersuchenden Kraftstoffs geleitet. Anschließend wurde der Kraftstoff mit einem Ölbad auf 140 °C erhitzt und weitere 5 h bei dieser Temperatur gerührt. Nach Abkühlen auf Raumtemperatur wurde die gesamte Kraftstoffmenge über einen 0,45 µm Membranfilter filtriert. Anschließend wurde der Filterrückstand nach 45 min Trocknen im Trockenschrank bei 115 °C und anschließender 2-stündiger Trocknung unter Vakuum im Exsiccator gravimetrisch bestimmt:
Blindwert (ohne Additiv): 8,9 mg
erfindungsgemäß additiviert mit jeweils 100 mg/l der folgenden Verbindungen:

| | |
|---|---|
| Verbindung der Formel Vb (Herstellungsbeispiel 1): | 1,8 mg |
| Verbindung der Formel Ve (Herstellungsbeispiel 2): | 1,0 mg |
| Verbindung der Formel Vc (Herstellungsbeispiel 3): | 1,0 mg |
| Verbindung der Formel IIIv (Herstellungsbeispiel 4): | 1,6 mg |
| Verbindung der Formel IIIw (Herstellungsbeispiel 5): | 2,5 mg |
| Verbindung der Formel VIc (Herstellungsbeispiel 6): | 1,0 mg |

Durch den Einsatz des erfindungsgemäß verwendeten Additivs konnte die durch thermische Belastung des Turbinenkraftstoffs entstehende Partikelmenge deutlich reduziert werden.

### Beispiel 9: Verbesserung der thermischen Stabilität von Turbinenkraftstoff (jet fuel)

Es wurde ein Turbinenkraftstoff in Anlehnung an die Spezifikation Jet A-1 nach DEF STAN 91-91 bzw. ASTM D 1655 eingesetzt. Die Überprüfung der Thermostabilität erfolgte nach der JFTOT-Breakpoint-Methode nach ASTM D 3241. Bei dem nicht additivierten Turbinenkraftstoff wurde ein Wert von 250°C ermittelt. Mit Kraftstoffen, die mit jeweils 100 mg/l eines nachfolgend aufgeführten erfindungsgemäß verwendeten Additivs additiviert waren, wurden die folgenden Werte gemessen:

| | |
|---|---|
| Verbindung der Formel Vb (Herstellungsbeispiel 1): | 280°C |
| Verbindung der Formel Ve (Herstellungsbeispiel 2): | 260°C |
| Verbindung der Formel Vc (Herstellungsbeispiel 3): | 280°C |

### Beispiel 10: Überprüfung der Wasserverträglichkeit von Turbinenkraftstoff

Es wurden die Verbindung der Formel Vb aus Herstellungsbeispiel 1 und der gleiche Turbinenkraftstoff wie in Beispiel 8 und 9 verwendet.

Gemäß DIN 51415 bzw. ASTM D 1094 wurde nach Zugabe von 100 mg/l der Verbindung der Formel Vb die Wasserverträglichkeit des Turbinenkraftstoffes und damit die unerwünschte Neigung, Emulsionen zu bilden, bestimmt. Hierzu wurden 80 ml des additivierten Turbinenkraftstoffes und 20 ml Wasser in definierter Weise intensiv geschüttelt. Danach erfolgte eine visuelle Bewertung der Phasentrennschichten nach jeweils 1, 5, 30 und 60 Minuten. Bereits 5 Minuten nach Wasserzugabe erhielt man eine vollständige Trennung von Kraftstoff und Wasser, es blieben keine Emulsionsanteile zurück.

### Beispiel 11: Überprüfung der Wasserabtrennungseigenschaften von Turbinenkraftstoff

Es wurde ein Turbinenkraftstoff der Spezifikation Jet A-1 nach DEF STAN 91-91 bzw. ASTM D 1655 eingesetzt. Die Überprüfung der Neigung von Turbinenkraftstoffen bezüglich ihrer Wasserabtrennungseigenschaften erfolgte nach ASTM D 3948 ("MSEP"-Test). Charakteristisch für diese Messungen ist die Verwendung eines Standard-Koaleszierfilters mit abschließender Trübungsmessung der Kraftstoffphase. Bei der Messung wurden die erfindungsgemäß verwendeten Additive in Kombination mit dem Antioxidans 2,6-Di-tert.-butyl-4-methylphenol ("BHT") und dem Metalldeaktivator N,N'-Disalicyliden-1,2-diaminopropan in einem hierfür üblichen Lösungsmittel geprüft. Die Dosierung der erfindungsgemäß verwendeten Additive betrug jeweils 215 mg/l (bezogen auf deren 100%igen Wirkstoffgehalt). Es wurden folgende Benotungen für das Trübungsverhalten ermittelt [relative Bewertungsskala von 0 (schlechteste Note) bis 100 (beste Note)] :
Blindwert (ohne Additiv): 98

| | |
|---|---|
| Verbindung der Formel Vb (Herstellungsbeispiel 1): | 97 |
| Verbindung der Formel Vc (Herstellungsbeispiel 3): | 98 |

Es traten keine Verschlechterungen im Vergleich mit nicht-additiviertem Turbinenkraftstoff auf.

## Patentansprüche

1. Verwendung von mehrkernigen phenolischen Verbindungen mit bis zu 20 Benzolkernen pro Molekül, welche durch Umsetzung eines Tetrahydrobenzoxazins der allgemeinen Formel I 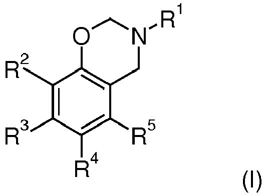
in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
in der die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit einem oder mehreren der gleichen oder verschiedener Phenole der allgemeinen Formel II 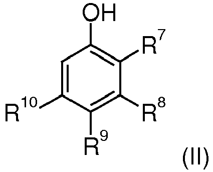 in der die Substituenten R⁷, R⁸, R⁹ und R¹⁰ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
und/oder mit einem oder mehreren der gleichen oder verschiedener Tetrahydrobenzoxazine der allgemeinen Formel I,
wobei der Substituent R⁴ auch für einen Rest der Formel Z und der Substituent R⁹ auch für einen Rest der Formel Z' stehen kann 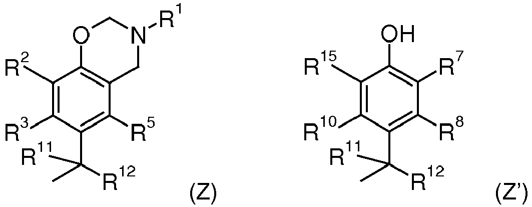
in denen die Substituenten R¹, R², R³, R⁵, R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR¹³-CH₂- auch einen zweiten Tetrahydrooxazin-Ring oder die Substituenten R² und R³ und R⁴ und R⁵ mit den an den Benzolkern angebundenen Teilstrukturen -O-CH₂-NR¹³-CH₂- und -O-CH₂-NR¹⁴-CH₂- auch einen zweiten und einen dritten Tetrahydrooxazin-Ring ausbilden können, wobei R¹³ und R¹⁴ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R¹, R⁸, R⁹, R¹⁰, R¹³ oder R¹⁴ 13 bis 3000 Kohlenstoffatome aufweist und die übrigen Substituenten aus der Gruppe R¹, R², R³, R⁴, R⁵, R⁷, R⁸, R⁹, R¹⁰, R¹³ oder R¹⁴, wenn sie für Hydrocarbylreste stehen, jeweils 1 bis 20 Kohlenstoffatome aufweisen,
erhältlich sind, als Dispergatoren zur Verhinderung von Ablagerungen im Kraftstoffsystem und/oder Verbrennungssystem von Turbinen.

2. Verwendung von mehrkernigen phenolischen Verbindungen nach Anspruch 1, bei denen mindestens einer der Substituenten R¹, R², R³, R⁴, R⁵, R⁷, R⁸, R⁹, R¹⁰, R¹³ oder R¹⁴ einen Polyisobutenyl-Rest mit einem zahlenmittleren Molekulargewicht Mₙ von 183 bis 42.000 darstellt.

3. Verwendung von mehrkernigen phenolischen Verbindungen nach Anspruch 2, bei denen ein oder zwei Polyisobutenyl-Reste mit einem zahlenmittleren Molekulargewicht Mₙ von 183 bis 42.000 im Molekül als Substituent R¹ und/oder R² und/oder R⁴ und/oder R⁷ und/oder R⁹ und/oder R¹³ und/oder R¹⁴ auftreten.

4. Verwendung von mehrkernigen phenolischen Verbindungen nach den Ansprüchen 1 bis 3, bei denen die übrigen Substituenten aus der Gruppe R¹, R², R³, R⁴, R⁵, R⁷, R⁸, R⁹, R¹⁰, R¹³ und R¹⁴, die nicht für Substituenten mit 13 bis 3000 Kohlenstoffatomen bzw. für Polyisobutenyl-Reste mit einem zahlenmittleren Molekulargewicht Mₙ von 183 bis 42.000 stehen, unabhängig voneinander Wasserstoffatome, Hydroxylgruppen oder, wenn sie für Hydrocarbylreste stehen, lineare oder verzweigte C₁- bis C₄-Alkylreste bezeichnen.

5. Verwendung von mehrkernigen phenolischen Verbindungen nach den Ansprüchen 1 bis 4 mit einem zahlenmittleren Molekulargewicht Mₙ von 285 bis 25.000.

6. Verwendung von mehrkernigen phenolischen Verbindungen nach Anspruch 2 oder 3, wobei der gemittelte Polydispersitätsindex PDI für die Polyisobutenyl-Reste im den mehrkernigen phenolischen Verbindungen höchstens das 5-fache des gemittelten Polydispersitätsindex PDI für die Polyisobutenyl-Reste in den zugrundeliegenden Tetrahydrobenzoxazinen I und/oder Phenolen II beträgt.

7. Turbinenkraftstoffzusammensetzung, enthaltend einen Turbinenkraftstoff (jet fuel) und wenigstens eine mehrkernige phenolische Verbindung, wie in einem der Ansprüche 1 bis 6 definiert.

8. Turbinenkraftstoffzusammensetzung nach Anspruch 7, **dadurch gekennzeichnet, daß** der Turbinenkraftstoff ausgewählt ist aus der Gruppe bestehend aus Jet Fuel A, Jet Fuel A-1, Jet Fuel B, Jet Fuel JP-4, JP-5, JP-7, JP-8 und JP-8+100, Jet A und Jet A-1 gemäß ASTM D 1655 sowie DEF STAN 91-91.

9. Turbinenkraftstoffzusammensetzung nach Anspruch 7 oder 8, **dadurch gekennzeichnet, daß** die wenigstens eine mehrkernige phenolische Verbindung in einer in einer Menge von 0,0001 bis 1 Gew.-% anwesend ist, bezogen auf die Gesamtmenge der Turbinenkraftstoffzusammensetzung.

## Claims

1. The use of polycyclic phenolic compounds which have up to 20 benzene rings per molecule and are obtainable by reacting a tetrahydrobenzoxazine of the general formula I 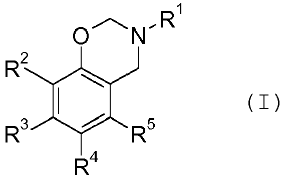
in which the substituent R¹ is a hydrocarbyl radical which has from 1 to 3000 carbon atoms and may be interrupted by one or more heteroatoms from the group of O and S and/or by one or more NR⁶ moieties,
where R⁶ is a hydrogen atom or a C₁- to C₄-alkyl radical, and
in which the substituents R², R³, R⁴ and R⁵ are each independently hydrogen atoms, hydroxyl groups or hydrocarbyl radicals which have in each case from 1 to 3000 carbon atoms and may be interrupted by one or more heteroatoms from the group of O and S and/or by one or more NR⁶ moieties, where R⁶ is as defined above,
with one or more of the same or different phenols of the general formula II 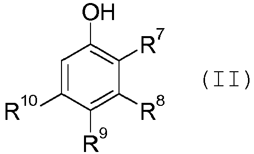 in which the substituents R⁷, R⁸, R⁹ and R¹⁰ are each independently hydrogen atoms, hydroxyl groups or hydrocarbyl radicals which have in each case from 1 to 3000 carbon atoms and may be interrupted by one or more heteroatoms from the group of O and S and/or by one or more NR⁶ moieties, where R⁶ is as defined above,
and/or with one or more of the same or different tetrahydrobenzoxazines of the general formula I,
where the substituent R⁴ may also be a radical of the formula Z and the substituent R⁹ may also be a radical of the formula Z' 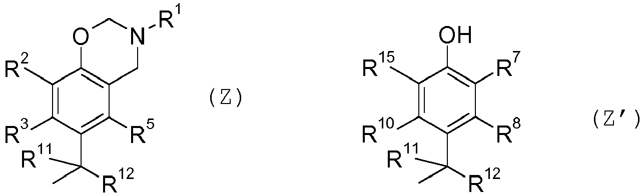
in which the substituents R¹, R², R³, R⁵, R⁷, R⁸ and R¹⁰ are each as defined above, the substituent R⁷ may also be a radical derived from a tetrahydrobenzoxazine of the general formula I, the substituent R¹⁵ is hydrogen or a radical derived from a tetrahydrobenzoxazine of the general formula I, and the substituents R¹¹ and R¹² may be the same or different and are each hydrogen or a C₁- to C₁₀-alkyl radical,
and in which the substituents R² and R³ or R³ and R⁴ or R⁴ and R⁵, together with the substructure -O-CH₂-NR¹³-CH₂- attached to the benzene ring, may also form a second tetrahydrooxazine ring, or the substituents R² and R³ and R⁴ and R⁵, together with the substructures - O-CH₂-NR¹³-CH₂- and -O-CH₂-NR¹⁴-CH₂- attached to the benzene ring, may also form a second and a third tetrahydrooxazine ring, where R¹³ and R¹⁴ are each independently hydrocarbyl radicals which have in each case from 1 to 3000 carbon atoms and may be interrupted by one or more heteroatoms from the group of O and S and/or by one or more NR⁶ moieties, where R⁶ is as defined above,
with the proviso that at least one of the substituents R¹, R², R³, R⁴, R⁵, R⁷, R⁸, R⁹, R¹⁰, R¹³ or R¹⁴ has from 13 to 3000 carbon atoms, and the remaining substituents from the group of R¹, R², R³, R⁴, R⁵, R⁷, R⁸, R⁹, R¹⁰, R¹³ or R¹⁴, when they are hydrocarbyl radicals, each have from 1 to 20 carbon atoms
as dispersants for preventing deposits in the fuel system and/or combustion system of turbines.

2. The use of polycyclic phenolic compounds according to claim 1, in which at least one of the substituents R¹, R², R³, R⁴, R⁵, R⁷, R⁸, R⁹, R¹⁰, R¹³ or R¹⁴ is a polyisobutenyl radical having a number-average molecular weight Mₙ of from 183 to 42 000.

3. The use of polycyclic phenolic compounds according to claim 2, in which one or two polyisobutenyl radicals having a number-average molecular weight Mₙ of from 183 to 42 000 occur in the molecule as substituent R¹ and/or R² and/or R⁴ and/or R⁷ and/or R⁹ and/or R¹³ and/or R¹⁴.

4. The use of polycyclic phenolic compounds according to claims 1 to 3, in which the remaining substituents from the group of R¹, R², R³, R⁴, R⁵, R⁷, R⁸, R⁹, R¹⁰, R¹³ and R¹⁴ which are not substituents having from 13 to 3000 carbon atoms or polyisobutenyl radicals having a number-average molecular weight Mₙ of from 183 to 42 000 are each independently hydrogen atoms, hydroxyl groups or, when they are hydrocarbyl radicals, linear or branched C₁- to C₄-alkyl radicals.

5. The use of polycyclic phenolic compounds according to claims 1 to 4 and having a number-average molecular weight Mₙ of from 285 to 25 000.

6. The use of polycyclic phenolic compounds according to claim 2 or 3, wherein the mean polydispersity index PDI for the polyisobutenyl radicals in the polycyclic phenolic compounds is at most 5 times the mean polydispersity index/PDI for the polyisobutenyl radicals in the parent tetrahydrobenzoxazines I and/or phenols II.

7. A turbine fuel composition comprising a turbine fuel (jet fuel) and at least one polycyclic phenolic compound as defined in any of claims 1 to 6.

8. The turbine fuel composition according to claim 7, wherein the turbine fuel is selected from the group consisting of Jet Fuel A, Jet Fuel A-1, Jet Fuel B, Jet Fuel JP-4, JP-5, JP-7, JP-8 and JP-8+100, Jet A and Jet A-1 according to ASTM D 1655 and DEF STAN 91-91.

9. The turbine fuel composition according to claim 7 or 8, wherein the at least one polycyclic phenolic compound is present in an amount of from 0.0001 to 1 % by weight, based on the total amount of the turbine fuel composition.

## Revendications

1. Utilisation de composés phénoliques polynucléaires contenant jusqu'à 20 noyaux benzène par molécule, qui peuvent être obtenus par mise en réaction d'une tétrahydrobenzoxazine de formule générale I 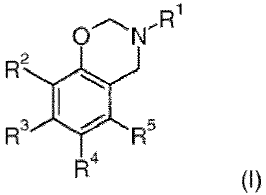
dans laquelle le substituant R¹ désigne un radical hydrocarbyle de 1 à 3 000 atomes de carbone, qui peut être interrompu par un ou plusieurs hétéroatomes du groupe constitué par O et S et/ou par un ou plusieurs groupes NR⁶,
R⁶ désignant un atome d'hydrogène ou un radical alkyle en C₁ à C₄, et
dans laquelle les substituants R², R³, R⁴ et R⁵ représentent indépendamment les uns des autres des atomes d'hydrogène, des groupes hydroxyle ou des radicaux hydrocarbyle contenant chacun 1 à 3 000 atomes de carbone, qui peuvent être interrompus par un ou plusieurs hétéroatomes du groupe constitué par O et S et/ou par un ou plusieurs groupes NR⁶, R⁶ ayant la signification indiquée précédemment,
avec un ou plusieurs phénols identiques ou différents de formule générale II 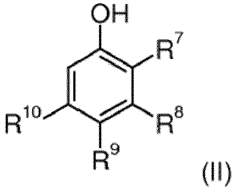 dans laquelle les substituants R⁷, R⁸, R⁹ et R¹⁰ représentent indépendamment les uns des autres des atomes d'hydrogène, des groupes hydroxyle ou des radicaux hydrocarbyle contenant chacun 1 à 3 000 atomes de carbone, qui peuvent être interrompus par un ou plusieurs hétéroatomes du groupe constitué par O et S et/ou par un ou plusieurs groupes NR⁶, R⁶ ayant la signification indiquée précédemment,
et/ou avec une ou plusieurs tétrahydrobenzoxazines identiques ou différentes de formule générale I,
le substituant R⁴ pouvant également représenter un radical de formule Z et le substituant R⁹ pouvant également représenter un radical de formule Z', 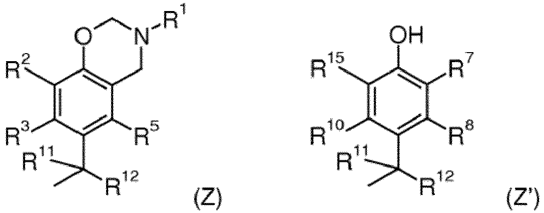
dans lesquelles les substituants R¹, R², R³, R⁵, R⁷, R⁸ et R¹⁰ ont les significations susmentionnées, le substituant R⁷ peut également être un radical dérivé d'une tétrahydrobenzoxazine de formule générale I, le substituant R¹⁵ représente l'hydrogène ou un radical dérivé d'une tétrahydrobenzoxazine de formule générale I, et les substituants R¹¹ et R¹² peuvent être identiques ou différents, et désignent l'hydrogène ou un radical alkyle en C₁ à C₁₀,
et dans lesquelles les substituants R² et R³ ou R³ et R⁴ ou R⁴ et R⁵ peuvent également former avec la structure partielle -O-CH₂-NR¹³-CH₂- reliée au noyau benzène un deuxième cycle tétrahydrooxazine, ou les substituants R² et R³ et R⁴ et R⁵ peuvent également former avec les structures partielles -O-CH₂-NR¹³-CH₂- et -O-CH₂-NR¹⁴-CH₂-reliées au noyau benzène un deuxième et un troisième cycle tétrahydrooxazine, R¹³ et R¹⁴ signifiant indépendamment l'un de l'autre des radicaux hydrocarbyle contenant chacun 1 à 3 000 atomes de carbone, qui peuvent être interrompus par un ou plusieurs hétéroatomes du groupe constitué par O et S et/ou par un ou plusieurs groupes NR⁶, R⁶ ayant la signification indiquée précédemment,
à condition qu'au moins un des substituants R¹, R², R³, R⁴, R⁵, R⁷, R⁸, R⁹, R¹⁰, R¹³ ou R¹⁴ comprenne 13 à 3 000 atomes de carbone et que les autres substituants du groupe constitué par R¹, R², R³, R⁴, R⁵, R⁷, R⁸, R⁹, R¹⁰, R¹³ ou R¹⁴ comprennent chacun, lorsqu'ils représentent des radicaux hydrocarbyle, 1 à 20 atomes de carbone,
en tant que dispersants pour l'inhibition de dépôts dans le système de carburant et/ou le système de combustion de turbines.

2. Utilisation de composés phénoliques polynucléaires selon la revendication 1, dans laquelle au moins un des substituants R¹, R², R³, R⁴, R⁵, R⁷, R⁸, R⁹, R¹⁰, R¹³ ou R¹⁴ représente un radical polyisobutényle ayant un poids moléculaire moyen en nombre Mₙ de 183 à 42 000.

3. Utilisation de composés phénoliques polynucléaires selon la revendication 2, dans laquelle un ou deux radicaux polyisobutényle ayant un poids moléculaire moyen en nombre Mₙ de 183 à 42 000 sont présents dans la molécule en tant que substituant R¹ et/ou R² et/ou R⁴ et/ou R⁷ et/ou R⁹ et/ou R¹³ et/ou R¹⁴.

4. Utilisation de composés phénoliques polynucléaires selon les revendications 1 à 3, dans laquelle les autres substituants du groupe constitué par R¹, R², R³, R⁴, R⁵, R⁷, R⁸, R⁹, R¹⁰, R¹³ et R¹⁴, qui ne représentent pas des substituants contenant 13 à 3 000 atomes de carbone ou des radicaux polyisobutényle ayant un poids moléculaire moyen en nombre Mₙ de 183 à 42 000, désignent indépendamment les uns des autres des atomes d'hydrogène, des groupes hydroxyle ou, lorsqu'ils représentent des radicaux hydrocarbyle, des radicaux alkyle en C₁ à C₄ linéaires ou ramifiés.

5. Utilisation de composés phénoliques polynucléaires selon les revendications 1 à 4, ayant un poids moléculaire moyen en nombre Mₙ de 285 à 25 000.

6. Utilisation de composés phénoliques polynucléaires selon la revendication 2 ou 3, dans laquelle l'indice de polydispersité moyen PDI pour les radicaux polyisobutényle dans les composés phénoliques polynucléaires est d'au plus 5 fois l'indice de polydispersité moyen PDI pour les radicaux polyisobutényle dans les tétrahydrobenzoxazines I et/ou les phénols II sous-jacents.

7. Composition de carburant pour turbine, contenant un carburant pour turbine (jet fuel) et au moins un composé phénolique polynucléaire, tel que défini dans l'une quelconque des revendications 1 à 6.

8. Composition de carburant pour turbine selon la revendication 7, **caractérisée en ce que** le carburant pour turbine est choisi dans le groupe constitué par Jet Fuel A, Jet Fuel A-1, Jet Fuel B, Jet Fuel JP-4, JP-5, JP-7, JP-8 et JP-8+100, Jet A et Jet A-1 selon ASTM D 1655, ainsi que DEF STAN 91-91.

9. Composition de carburant pour turbine selon la revendication 7 ou 8, **caractérisée en ce que** ledit au moins un composé phénolique polynucléaire est présent en une quantité de 0,0001 à 1 % en poids, par rapport à la quantité totale de la composition de carburant pour turbine.
